# EUROPÄISCHE PATENTSCHRIFT

(11) **EP 1 984 532 B1**
(45) Veröffentlichungstag und Bekanntmachung des Hinweises auf die Patenterteilung: **12.05.2010**
(21) Anmeldenummer: 07704060.8
(22) Anmeldetag: 22.01.2007
(51) Int. Cl.: C22B 11/08, C01C 3/08, C22B 3/44

(54) **BEREITSTELLUNG VON METALLCYANID ENTHALTENDEN WÄSSRIGEN LÖSUNGEN FÜR DIE CYANIDLAUGEREI ZUR GOLD- UND SILBERGEWINNUNG**
PROVISION OF METAL CYANIDE CONTAINING AQUEOUS SOLUTIONS FOR GOLD AND SILVER WINNING BY CYANIDE LEACHING
PRÉPARATION DE SOLUTIONS AQUEUSES À CYANURE DE MÉTAL POUR L'EXTRACTION DE L'OR ET DE L'ARGENT PAR LIXIVIATION CYANIDIQUE

(30) Priorität: 03.02.2006 EP 06101295
(43) Veröffentlichungstag der Anmeldung: 29.10.2008
(73) Patentinhaber: BASF SE, 67056 Ludwigshafen (DE)
(72) Erfinder: DECKERS, Andreas, 55234 Flomborn (DE); SCHNEIDER, Thomas, 67227 Frankenthal (DE)
(86) Internationale Anmeldenummer: PCT/EP2007/050592
(87) Internationale Veröffentlichungsnummer: WO 2007/090725

(56) Entgegenhaltungen:
- US-A- 3 619 132
- US-A- 4 687 559
- US-A- 4 721 526
- US-A- 4 847 062
- US-A- 5 254 153
- US-A- 5 368 830

## Beschreibung

Die vorliegende Erfindung betrifft ein Verfahren zur Herstellung von Lösungen enthaltend Wasser und mindestens ein Metallcyanid, wobei mindestens ein Teil des Wassers aus Abwasser gewonnen wird, das in einem Verfahren zur Gewinnung von Edelmetallen aus edelmetallhaltigen Erzen durch Cyanidlaugerei als abgereichertes Abwasser anfällt.

Ein wichtiger Einsatzbereich für wässrige cyanidhaltige Lösungen ist die Cyanidlaugerei von Edelmetallen aus edelmetallhaltigen Erzen. Das Verfahren wird im Allgemeinen zur Gewinnung von Edelmetallen aus Erzen eingesetzt, die geringe Anteile an Edelmetall enthalten, so dass eine Gewinnung der Edelmetalle gemäß anderen im Stand der Technik bekannten Verfahren nicht ökonomisch ist.

Bei der Cyanidlaugerei wird üblicherweise zerkleinertes und gegebenenfalls agglomeriertes edelmetallhaltiges Erz mit einer wässrigen cyanidhaltigen Lösung in Anwesenheit von Sauerstoff behandelt. Dabei wird die Bildung eines Edelmetall-Cyanid-Komplexes ausgenutzt, um das im Erz vorhandene Edelmetall von anderen Metallen zu trennen. Dieses Verfahren wird im Allgemeinen zur Gewinnung von Silber und Gold, aus silber- und goldhaltigen Erzen eingesetzt. Im Folgenden ist die Bildung eines Gold-Cyanid-Komplexes beispielhaft dargestellt:

2 Au + H₂O + ½ O₂ + 4 KCN → 2 K[Au(CN)₂] + 2 KOH

Das Edelmetall - im vorliegenden Fall das Gold - liegt nach der Behandlung des Erzes mit der wässrigen cyanidhaltigen Lösung in Anwesenheit von Sauerstoff komplexiert in wässriger Lösung vor. Anschließend wird die goldhaltige Lösung im Allgemeinen von den Erzrückständen abgetrennt und das Gold zum Beispiel durch Fällung mit Zinkstaub gewonnen:

2 K[Au(CN)₂] + Zn → K₂[Zn(CN)₄] + 2 Au

Neben der Ausfällung des Goldes mit Hilfe von Zinkstaub sind weitere Verfahren zur Gewinnung des Goldes im Stand der Technik bekannt, zum Beispiel die Adsorption mit Hilfe von Aktivkohle.

Im Anschluss an die vorstehend erwähnte Gewinnung des Edelmetalls, insbesondere des Goldes, durch Cyanidlaugerei bleibt ein an Edelmetallen abgereichertes Abwasser zurück, das Cyanid sowie gegebenenfalls Schwermetalle und weitere Feststoffe aufweist. Das an Edelmetallen abgereicherte Abwasser, das nicht abreagiertes toxisches Cyanid enthält, kann optional im Kreis gefahren werden, ohne dass der Cyanidgehalt aufgestockt wird, und wird auf diese Weise wiederum mit edelmetallhaltigem Erz in Kontakt gebracht. Am Ende des Prozesses zur Edelmetallgewinnung durch die Cyanidlaugerei fällt aber ein verunreinigtes und Cyanid-belastetes Abwasser an, das im Allgemeinen unter Aufwendung von hohen Kosten entsorgt werden muss.

Geeignete Verfahren zur Gewinnung von Edelmetallen mittels Cyanidlaugerei sind im Stand der Technik bekannt und zum Beispiel in Ullmann's Encyclopedia, 6th edition, 2000, Electronic Release, Kapitel Gold, Gold Alloys, and Gold Compounds, 4. Production, offenbart. Spezielle Verfahren zur Cyanidlaugerei, die insbesondere die Hafenlaugung betreffen, sind ebenfalls im Stand der Technik offenbart.

So betrifft DE-A 37 36 243 ein Verfahren und eine Anlage zur Gewinnung von Gold aus Golderz. Das Verfahren umfasst die Gewinnung von Gold aus Golderz durch Zerkleinern, Agglomerieren und Laugen, wobei das Erz in einem Arbeitsgang durch eine Beanspruchung im Gutbett zerkleinert und zugleich agglomeriert wird. Bezüglich einer Weiterverwendung des nach Rückgewinnung des Goldes an Gold abgereicherten Abwassers enthält DE-A 37 36 243 keinen Hinweis.

US 4,721,526 betrifft ein Verfahren zur Haufenlaugung von Gold- und Silbererzen, wobei die aus den Erzen gewinnbare Menge Gold und Silber dadurch erhöht wird, dass während der Cyanidlaugung ein Gas zugeleitet wird, das eine höhere Menge an Sauerstoff aufweist als Luft. Das Gold bzw. Silber wird aus der an Gold bzw. Silber angereicherten Lauge durch Zinkfällung oder Adsorption an Aktivkohle oder ähnliche Verfahren zurückgewonnen. Die an Gold oder Silber abgereicherte Lösung kann zur Haufenlaugung zurückgeführt werden. Wässrige Cyanidlösungen, worin ein Teil des Wassers aus Abwasser gewonnen wird, und deren Herstellungsverfahren sind in US 4,721,526 nicht offenbart.

US 5,368,830 betrifft ein Verfahren zur Cyanidlaugerei, wobei die Bildung und Ablagerung von Calciumcarbonat auf verschiedenen metallischen Oberflächen und Aktivkohle bzw. weiteren Oberflächen, die in Vorrichtungen zur Cyanidlaugerei vorliegen, vermieden wird. Die Vermeidung wird dadurch erreicht, dass den zur Cyanidlaugerei eingesetzten wässrigen Lösungen geringe Mengen von Polyetherpolyaminomethylenphosphonaten zugesetzt werden. Gemäß US 5,368,830 kann das bei der Cyanidlaugerei nach Gewinnung des Edelmetalls erhaltene abgereicherte Abwasser durch Zugabe von Cyanid und Calciumchlorid wieder "revitalisiert" werden und wiederum zur Cyanidlaugerei, insbesondere zur Haufenlaugung, eingesetzt werden. Lösungen enthaltend Wasser und mindestens ein Metallcyanid, wobei ein Teil des Wassers aus Abwasser gewonnen wird, und deren Herstellungsverfahren sind in US 5,368,830 nicht offenbart.

US 4,687,559 betrifft ein Verfahren zur Rückgewinnung von Wertstoffen aus metallurgischen Rückständen wie Anodenschlamm. Das Verfahren umfasst die Schritte (a) Aufschlämmen eines metallurgischen Rückstandes mit einer Extraktionslösung enthaltend Alkalimetallcyanid und Carbonatreagenzien in Anwesenheit von Wasserstoffperoxid, (b) Trennen der erhaltenen Lösung enthaltend Gold, Silber und/oder Selen von dem Rückstand, (c) Zurückgewinnung von Gold, Silber und/oder Selen aus der Lösung und Regenerieren des Cyanidreagenzes, (d) Trennung von Gold, Silber und/oder Selen und gelöste Verunreinigungen durch Ionenaustausch von dem Cyanidreagenz, (e) Bringen der Konzentration des regenerierten Cyanidreagenzes durch Alkalimetallcyanid- und Carbonatzugabe auf den gewünschten Gehalt, und (f) Zurückführen der Reagenzlösung aus Schritt (e) in Schritt (a), um frischen Rückstand zu extrahieren.

Die Herstellung der für die Cyanidlaugerei eingesetzten Cyanide ist ebenfalls aus dem Stand der Technik bekannt, und zum Beispiel in Ullmann's Encyclopedia, 6th Edition, 2000, Electronic Release, Kapitel Cyano Compounds, Inorganic, offenbart. Weitere Verfahren zur Herstellung von Alkalicyaniden, insbesondere Natriumcyanid sind zum Beispiel in US 2004/0197256 A1, US 3,619,132, US 4,847,062 und US 6,162,263 offenbart. Die Herstellung der Alkalicyanide erfolgt dabei durch Umsetzung von Blausäure-haltigen Gasen mit den entsprechenden Alkalilaugen.

So betrifft US 2004/0197256 A1 ein Verfahren zur Herstellung von Natriumcyanid durch Umsetzung von HCN mit wässriger NaOH, wobei eine Lösung enthaltend Natriumcyanid erhalten wird, und Kristallisation des Natriumcyanids, wobei eine Aufschlämmung von Natriumcyanid-Kristallen erhalten wird. Das Verfahren wird in Anwesenheit einer Säure oder eines Metallsalzes dieser Säure durchgeführt, wobei die Säure einen pKa-Wert von ≤ 4,8 aufweist. Die erhaltenen Natriumcyanid-Kristalle werden anschließend von der Aufschlämmung getrennt und getrocknet. Die erhaltenen Natriumcyanid-Kristalle können in der Cyanidlaugerei eingesetzt werden. Dazu werden die Kristalle im Allgemeinen in Wasser gemäß im Stand der Technik bekannten Verfahren gelöst. Die mit Hilfe des in US 2004/0197256 A1 hergestellten Natriumcyanid-Kristalle weisen eine hohe Reinheit auf. Sie sind gemäß US 2004/0197256 A1 besser für den Transport zum Anwender geeignet, als nach üblichen Verfahren hergestelltes Natriumcyanid in Form von Briketts. Des Weiteren ist der Transport der gemäß diesem Verfahren hergestellten Natriumcyanid-Kristalle preiswerter und weniger umweltgefährdend als der Transport von wässrigen Natriumcyanid-Lösungen.

US 3,619,132 betrifft ein Verfahren zur Herstellung von Alkalicyaniden durch Umsetzung eines Blausäure-enthaltenden Kohlendioxid-freien Rohgases mit wässrigem Alkalihydroxid in einem ersten Schritt bei einem Druck unterhalb von Atmosphärendruck, wobei das Alkalicyanid gebildet wird, und anschließende Kristallisation des Alkalicyanids in einem zweiten Schritt bei einem Druck unterhalb des Drucks im ersten Schritt.

Es werden Natriumcyanid-Kristalle erhalten, die anschließend getrocknet werden, wobei Natriumcyanid in hoher Reinheit erhalten wird.

US 4,847,062 betrifft ein Verfahren zur Herstellung von Natriumcyanid-Kristallen durch Umsetzung von Blausäure-enthaltendem Rohgas, das mittels des Andrussow-Prozesses hergestellt wird, enthaltend Oxide des Kohlenstoffs und Wassers, mit Natriumhydroxid. Aus der erhaltenen wässrigen Natriumcyanidlösung wird Natriumcyanid in Form von Kristallen isoliert. Die erhaltenen Natriumcyanid-Kristalle werden im Allgemeinen zu Briketts geformt und zum Anwender transportiert, der das Natriumcyanid anschließend wiederum in Wasser löst und die wässrige Lösung zum Beispiel zur Gewinnung von Metallen aus metallhaltigen Erzen einsetzt.

US 6,162,263 betrifft ein Verfahren zur Herstellung von Metallcyaniden, die für den Transport geeignet sind. Dabei werden die Metallcyanide in Form eines feuchten "Kuchens" erhalten.

Die bekannten Verfahren zur Herstellung von Cyaniden gehen davon aus, dass das Cyanid bei einem Hersteller außerhalb der Reichweite einer Erzmine produziert wird, in der die Cyanidlaugerei erfolgt. Das Cyanid wird dann als Feststoff oder gegebenenfalls als konzentrierte Lösung transportiert. In der Mine wird das Cyanid dann auf die für die Cyanidlaugerei notwendige Konzentration verdünnt. Die Verwendung von Abwasser, das bei der Cyanidlaugerei als abgereichertes Abwasser anfällt zur Herstellung von Cyanidlösungen wird im Stand der Technik nicht beschrieben.

Aufgabe der vorliegenden Erfindung ist somit die Bereitstellung eines Verfahrens zur Herstellung von Metallcyanid-enthaltenden wässrigen Lösungen, wobei mindestens ein Teil des Wassers aus Abwasser gewonnen wird, das bei der Cyanidlaugerei anfällt. Dadurch kann die bei der Cyanidlaugerei entstehende Abwassermenge verringert werden und es können Kosten für die Entsorgung des cyanidhaltigen Abwassers eingespart werden.

Gegenstand der vorliegenden Erfindung ist daher ein Verfahren zur Herstellung von Lösungen enthaltend Wasser und mindestens ein Metallcyanid umfassend:
(i) Umsetzung von Blausäure oder Blausäure enthaltenden Gasen mit einer Lösung enthaltend mindestens ein Metallhydroxid und/oder Metalloxid und/oder Mischoxid und Wasser;
(ii) gegebenenfalls Verdünnen der in Schritt (i) erhaltenen Lösung mit Wasser;
wobei in Schritt (i) mindestens ein Teil des Wassers aus Abwasser gewonnen wird, das in einem Verfahren zur Gewinnung von Edelmetallen aus edelmetallhaltigen Erzen durch Cyanidlaugerei als abgereichertes Abwasser anfällt.

Das bei der Cyanidlaugerei erhaltene abgereicherte Abwasser enthält üblicherweise Feststoffe und/oder Schwermetalle, zum Beispiel Zink. Diese Feststoffe werden bevorzugt vor dem Einsatz des Abwassers in den erfindungsgemäß hergestellten Lösungen abgetrennt, so dass in den erfindungsgemäß hergestellten Lösungen bevorzugt Abwasser eingesetzt wird, das ein von Feststoffen und/oder Schwermetallen geklärtes in der Cyanidlaugerei anfallendes abgereichertes Abwasser ist. Die Abtrennung der Feststoffe und/oder Schwermetalle aus dem in der Cyanidlaugerei anfallenden abgereicherten Abwasser erfolgt im Allgemeinen nach dem Fachmann bekannten Verfahren.

Die erfindungsgemäß hergestellten Lösungen enthalten optional neben dem Teil des Wassers, der aus dem vorstehend genannten Abwasser gewonnen wird, einen weiteren Anteil an Wasser, der durch Frischwasser gebildet wird. Bei diesem Frischwasser kann es sich um nicht besonders gereinigtes Wasser, z.B. Leitungswasser oder Flusswasser oder um gereinigtes Wasser, z.B. voll entsalztes (VE) Wasser, handeln.

Im Allgemeinen beträgt der Anteil des aus dem Abwasser gewonnenen Wassers 0,1 bis 100 Gew.-%, bevorzugt 5 bis 95 Gew.-%, besonders bevorzugt 10 bis 90 Gew.-%. Der Anteil des Frischwassers an dem in den erfindungsgemäß hergestellten Lösungen eingesetzten Wasser beträgt im Allgemeinen 0 bis 99,9 Gew.-%, bevorzugt 5 bis 95 Gew.-%, besonders bevorzugt 10 bis 90 Gew.-%. In einer bevorzugten Ausführungsform ist somit ein Teil der Wassers Frischwasser und ein Teil des Wassers wird aus Abwasser gewonnen.

Verfahren zur Gewinnung von Edelmetallen aus edelmetallhaltigen Erzen durch Cyanidlaugerei sind dem Fachmann bekannt. Eine Beschreibung des gesamten Verfahrens zur Cyanidlaugerei sowie inbesondere die Behandlung des Abwassers ist z.B. offenbart in: Technical Report "Treatment of Cyanide Heap Leaches and Tailings", September 1994, U.S. Environmental Protection Agency, Office of Solid Waste, Special Waste Branch, 401 M Street, SW, Washington, DC 20460.

Im Allgemeinen wird die Cyanidlaugerei zur Gewinnung von Silber oder Gold, insbesondere zur Gewinnung von Gold, eingesetzt. Dabei sind im Stand der Technik verschiedene Verfahren zur Laugung bekannt. So kann die Laugung als so genannte Haufenlaugung, Rührlaugung oder Sickerlaugung in Becken (Beckenlaugung) erfolgen. Gemäß der vorliegenden Erfindung wird bevorzugt ein abgereichertes Abwasser eingesetzt, das bei der Haufenlaugung erhalten wird. Geeignete Verfahren zur Haufenlaugung sind dem Fachmann bekannt.

Als Metallcyanide werden in den erfindungsgemäß hergestellten Lösungen bevorzugt Alkalimetall- oder Erdalkalimetallcyanide, besonders bevorzugt Li-, Na-, K- oder Ca-Cyanid, ganz besonders bevorzugt Na-Cyanid oder Ca-Cyanid eingesetzt.

Das Metallcyanid ist in den erfindungsgemäß hergestellten Lösungen bevorzugt in einer Menge von 0,001 bis 35 Gew.-%, bevorzugt 0,01 bis 30 Gew.-%, besonders bevorzugt 0,02 bis 10 Gew.-%, bezogen auf das Gesamtgewicht der Lösungen, enthalten. Diese Lösungen können - in Abhängigkeit von der Konzentration an Metallcyanid - direkt in der Cyanidlaugerei, bevorzugt in der so genannten Haufenlaugung, eingesetzt werden, oder vor dem Einsatz in der Cyanidlaugerei weiter verdünnt werden. Übliche Konzentrationen an Metallcyanid der für die Cyanidlaugerei geeigneten Lösungen sind dem Fachmann bekannt. Geeignete Gebrauchskonzentrationen an Metallcyanid betragen z.B. 10 bis 1000 ppm, bevorzugt 50 bis 500 ppm.

Bei den erfindungsgemäß hergestellten Lösungen handelt es sich üblicherweise um alkalische Lösungen. Bevorzugt weisen die erfindungsgemäß hergestellten Lösungen einen pH-Wert von 7 bis 13,5, besonders bevorzugt 8 bis 13, ganz besonders bevorzugt 9 bis 12,5 auf. Die Einstellung des pH-Wertes kann mit allen gängigen basischen Materialien erfolgen. Besonders sind NaOH, CaO, CA(OH)₂ oder Mischungen aus den genannten Stoffen wie z.B. Kalk oder gelöschter Kalk geeignet.

### Schritt (i)

Die in Schritt (i) eingesetzte Blausäure bzw. die in Schritt (i) eingesetzten Blausäure enthaltenden Gase können nach beliebigen, dem Fachmann bekannten Verfahren hergestellt werden. Geeignete Verfahren zur industriellen Herstellung von Blausäure sind zum Beispiel die Umsetzung von Kohlenwasserstoffen, insbesondere Methan, mit Ammoniak (Andrussow-Prozess, BMA-Verfahren). Sowohl bei dem Andrussow-Prozess als auch bei dem BMA-Verfahren ist der Einsatz eines Edelmetallkatalysators erforderlich.

Eine weitere Möglichkeit zur Herstellung von Blausäure ist die Dehydratisierung von Formamid. Dabei werden im Allgemeinen aus Synthesegas (CO/H₂) zunächst Methanol und Methylformiat hergestellt, wobei das Methylformiat dann mit Ammoniak zu Formamid umamidiert wird. Das Formamid ist thermisch labil und zerfällt bei hohen Temperaturen zu Blausäure und Wasser. Die Spaltung ist sehr selektiv. Auf diese Weise ist ein Spaltgas erhältlich, das hohe Konzentrationen an Blausäure aufweist sowie lediglich geringe Mengen Ammoniak oder anderer gasförmiger Stoffe, wie CO₂, CO oder H₂. Des Weiteren ist in dem Verfahren zur Herstellung von Blausäure durch Dehydratisierung von Formamid kein teurer Edelmetallkatalysator erforderlich, und das Verfahren ist apparativ wenig aufwendig. Geeignete Verfahren zur Herstellung von Blausäure durch Dehydratisierung von Formamid sind zum Beispiel in EP-A 0 209 039, DE-A 101 385 53 und WO 2004/050587 genannt.

Die Herstellung von Blausäure bzw. von Blausäure enthaltenden Gasen in Schritt (i) des erfindungsgemäßen Verfahrens erfolgt bevorzugt durch Dehydratisierung von Formamid, insbesondere gemäß einem der vorstehend genannten Verfahren, da das hochsiedende Formamid einfach und sicher transportiert werden kann und in einfachen kostengünstigen Thermolyseanlage im Einzugsbereich einer Edelmetallmine zur Herstellung von HCN eingesetzt werden kann. Besonders bevorzugt erfolgt die Herstellung von Blausäure durch katalytische Dehydratisierung von gasförmigem Formamid in einem Reaktor, der eine innere Reaktoroberfläche aus Stahl enthaltend Eisen sowie Chrom und Nickel aufweist, wobei es sich bei dem Reaktor ganz besonders bevorzugt um einen Rohrreaktor handelt, der keine weiteren Einbauten enthält. Ein geeigneter Reaktor sowie ein geeignetes Verfahren sind zum Beispiel in WO 2004/050587 offenbart.

Zur Umsetzung mit einer Lösung enthaltend mindestens ein Metallhydroxid und/oder Metalloxid und/oder Mischoxid und Wasser kann die Blausäure gasförmig, insbesondere als gasförmige Mischung, enthaltend Blausäure, oder flüssig eingesetzt werden. Geeignete Verfahren zur Herstellung von Metallcyaniden durch Umsetzung von Blausäure mit einer Lösung enthaltend mindestens ein Metallhydroxid und/oder Metalloxid und/oder Mischoxid und Wasser sind dem Fachmann bekannt und wurden bereits vorstehend erwähnt.

In einer bevorzugten Ausführungsform erfolgt die Umsetzung in Schritt (i) durch Umsetzung eines Blausäure enthaltenden Rohgases, das besonders bevorzugt durch Dehydratisierung von Formamid erhalten wird, mit der Lösung enthaltend mindestens ein Metallhydroxid und/oder Metalloxid und/oder Mischoxid und Wasser. Das Blausäure-Rohgas kann vor der Umsetzung mit der Lösung enthaltend mindestens ein Metallhydroxid und/oder Metalloxid und/oder Mischoxid und Wasser von in geringen Mengen als Nebenprodukt bei der Blausäure-Herstellung entstehendem Ammoniak durch Säurewäsche befreit werden. Als Säure kann dabei jede mineralische Säure eingesetzt werden, wobei Schwefel- oder Phosphorsäure bevorzugt sind. Wird die Blausäure durch Dehydratisierung von Formamid hergestellt, was bevorzugt ist, ist eine Säurewäsche im Allgemeinen nicht erforderlich, da lediglich sehr geringe Mengen Ammoniak als Nebenprodukt bei der Herstellung von Blausäure entstehen. Geeignete Verfahren zur Säurewäsche sind zum Beispiel in Ullmann's Encyclopedia of Industrial Chemistry, 6th Edition, Kapitel HCN, offenbart.

Im Allgemeinen wird Schritt (i) des erfindungsgemäßen Verfahrens so durchgeführt, dass das gegebenenfalls mit Säure behandelte Rohgas enthaltend Blausäure in eine wässrige Lösung geleitet wird, die mindestens ein Metallhydroxid und/oder Metalloxid und/oder Mischoxid enthält.

Bevorzugt eingesetzte Metallhydroxide und/oder Metalloxide und/oder Mischoxide sind Alkalimetall- oder Erdalkalimetallhydroxide und/oder Erdalkalimetallhydroxide und/oder Erdalkalimetalloxide und/oder Erdalkalimetallmischoxide, besonders bevorzugt Li-, Na-, K- oder Ca-hydroxid und/oder Ca-Oxid und/oder Ca-Mischoxid, ganz besonders bevorzugt Na-Hydroxid oder Ca-Hydroxid und/oder Ca-Oxid und/oder Mischoxid. Bei dem Ca-Hydroxid und/oder Ca-Oxid und/oder Ca-Mischoxid kann es sich z. B. um ungelöschten Kalk oder gelöschten Kalk, Ca-O, Ca(OH)₂ handeln. In Abhängigkeit von dem eingesetzten Metallhydroxid und/oder Metalloxid und/oder Mischoxid wird das entsprechende Metallcyanid gebildet. Bei dem ganz besonders bevorzugten Einsatz von Na-Hydroxid oder Ca-Hydroxid und/oder Ca-Oxid und/oder Ca-Mischoxid wird somit Na-Cyanid oder Ca-Cyanid erhalten.

Im Allgemeinen enthält die Lösung enthaltend mindestens ein Metallhydroxid und Wasser 5 bis 50 Gew.-%, bevorzugt 15 bis 50 Gew.-%, besonders bevorzugt 30 bis 50 Gew.-% des eingesetzten Metallhydroxids und/oder Metalloxids und/oder Mischoxids.

Die Reaktionstemperatur in Schritt (i) beträgt im Allgemeinen 5 bis 100 °C, bevorzugt 10 bis 80 °C, besonders bevorzugt 20 bis 60 °C.

Üblicherweise wird solange Blausäure enthaltendes Gas in die Lösung enthaltend mindestens ein Metallhydroxid und/oder Metalloxid und/oder Mischoxid und Wasser eingeleitet, bis ein Überschuss an Hydroxid und/oder Oxid und/oder Mischoxid von im Allgemeinen 0,1 bis 5 Gew.-%, bevorzugt 0,2 bis 2,0 Gew.-%, errreicht wird. Wenn der genannte Überschuss an Hydroxid und/oder Oxid und/oder Mischoxid erreicht ist, wird die Einleitung des Blausäure enthaltenden Gases unterbrochen.

Es wird eine Lösung des gewünschten Blausäuresalzes in Wasser erhalten. Der Gehalt an Blausäuresalzen in der Lösung ist dabei abhängig von der eingesetzten Menge an Hydroxid und/oder Oxid und/oder Mischoxid in der Lösung.

Im Allgemeinen wird eine Lösung erhalten, die einen Gehalt an gewünschtem Blausäuresalz von 0,001 bis 35 Gew.-%, bevorzugt 0,01 bis 30 Gew.-%, besonders bevorzugt 0,02 bis 10 Gew.-% aufweist.

Ein bevorzugtes Verfahren zur Herstellung von Lösungen enthaltend Wasser und mindestens ein Metallcyanid ist zum Beispiel in der älteren nicht-vorveröffentlichten Anmeldung vom 7. Juni 2005 mit dem Aktenzeichen DE 10 2005 026 326.7 und dem Titel "Verfahren zur Herstellung von Salzen der Blausäure" offenbart.

Die in Schritt (i) erhaltene Lösung enthaltend mindestens ein Metallcyanid und Wasser kann in einem weiteren Schritt auf die gewünschte Gebrauchskonzentration von im Allgemeinen 10 bis 1000 ppm, bevorzugt 50 bis 500 ppm -verdünnt werden, falls die in Schritt (i) erhaltene Lösung das Metallcyanid in höherer Konzentration enthält.

### Schritt (ii)

In Schritt (ii), der optional ist, erfolgt eine Verdünnung der in Schritt (i) des erfindungsgemäßen Verfahrens erhaltenen Lösung mit Wasser. Dabei werden gebrauchsfertige Lösungen enthaltend Wasser und mindestens ein Metallcyanid erhalten, die direkt in der Cyanidlaugerei eingesetzt werden können.

Grundsätzlich ist es möglich, das gesamte erfindungsgemäße Verfahren umfassend Schritt (i) und Schritt (ii) in der Erzmine durchzuführen. Es ist jedoch ebenfalls denkbar, Schritt (i) des erfindungsgemäßen Verfahrens an einem anderen Standort als dem der Erzmine durchzuführen und lediglich eine Verdünnung in Schritt (ii) am Ort der Erzmine durchzuführen.

In einer weiteren Ausführungsform ist es möglich, nur Schritt (i) durchzuführen, wobei direkt eine gebrauchsfertige Lösung mit den vorstehend genannten Konzentrationen an dem mindestens einen Metallcyanid erhalten wird. Eine Verdünnung gemäß Schritt (ii) kann in diesem Fall entfallen.

Erfindungsgemäß wird in Schritt (i) mindestens ein Teil des Wassers aus Abwasser gewonnen, das in einem Verfahren zur Gewinnung von Edelmetallen aus edelmetallhaltigen Erzen durch Cyanidlaugerei als abgereichertes Abwasser anfällt. Dabei ist es möglich, dass das Abwasser zur Herstellung der in Schritt (i) eingesetzten Lösung enthaltend mindestens ein Metallhydroxid und/oder Metalloxid und/oder Mischoxid und Wasser eingesetzt wird. Dabei kann das Abwasser bereits zum Auflösen des Metallhydroxids eingesetzt werden oder lediglich zur Verdünnung der Lösung enthaltend mindestens ein Metallhydroxid und/oder Metalloxid und/oder Mischoxid und Wasser auf die gewünschte Konzentration an Metallhydroxid und/oder Metalloxid und/oder Mischoxid. Es ist ebenfalls möglich, sowohl in Schritt (i) als auch in Schritt (ii) Abwasser einzusetzen. Falls Schritt (ii) nicht durchgeführt wird, wird das Abwasser in Schritt (i) eingesetzt. In einer bevorzugten Ausführungsform des erfindungsgemäßen Verfahrens wird ein Teil des zur Herstellung der erfindungsgemäßen Lösungen enthaltend Wasser und mindestens ein Metallcyanid erforderlichen Wassers von Frischwasser gebildet. Dies ist vorteilhaft, um ggf. mögliche Aufpegelungen von Fremdstoffen zu vermeiden, die dadurch zustande kommen können, dass das bei der Cyanidlaugerei anfallende abgereicherte Wasser im Kreis gefahren wird. Üblicherweise erfolgt die bevorzugte Zugabe von Frischwasser in der Form, dass ein Purge-Strom des in der Cyanidlaugerei angefallenen abgereicherten Abwassers kontinuierlich gegen Frischwasser ersetzt wird.

Geeignete Anteile von Frischwasser und Abwasser in den erfindungsgemäß hergestellten Lösungen wurden bereits vorstehend genannt.

Die gemäß dem erfindungsgemäßen Verfahren hergestellten Lösungen enthaltend Wasser und mindestens ein Metallcyanid werden bevorzugt in einem Verfahren zur Gewinnung von Edelmetallen aus edelmetallhaltigen Erzen durch Cyanidlaugerei eingesetzt.

Die nachfolgenden Beispiele erläutern die Erfindung zusätzlich.

### Beispiele:

### Herstellung eines Rohgases enthaltend Blausäure

Ein 4,5 m langes Reaktionsrohr aus 1.4541-Stahl (V2A-Stahl) mit einem Innendurchmesser von 10 mm und einem Außendurchmesser von 12 mm wird elektrisch auf eine konstante Außentemperatur von 520°C gebracht. Das Reaktionsrohr besitzt eine spezifische Oberfläche von 400 m²/m³. Der Innendruck im Rohr beträgt 100 mbar abs. und wird durch eine Vakuumpumpe erzeugt.

In einem vorgeschalteten Verdampfer, der ebenfalls unter dem Reaktionsdruck steht, werden 1,3 kg/h Formamid bei 145°C verdampft und auf den Kopf des Reaktionsrohres geleitet. Zusätzlich wird an der Verbindung zwischen Verdampfer und Reaktionsrohr 13 NL Luft/h eingespeist.

Am Ende des Reaktionsrohres wird eine Probe genommen und diese auf ihre Bestandteile analysiert. Die Analyse ergab einen Umsatz des Formamids von 98,5 % und eine Blausäureselektivität bezogen auf Formamid von 93,2 %.

### 1 Herstellung von Blausäuresalz-Lösungen (Vergleich)

Prozessführung zur Herstellung des Blausäure enthaltenden Rohgases: Beispiel A1 (Formamid-Umsatz: 98,5 %; Blausäureselektivität: 93,2 %).

Die Zusammensetzung des in Beispiel A1 erhaltenen Rohgases ist wie folgt (in Gew.-%): 55,5 % HCN; 38,0 % Wasser; 1,5 % Formamid; 1,7 % NH₃; 2,9 % CO₂; 0,2 % H₂; 0,2 CO.

Zur NH₃-Entfernung wird das Rohgas durch eine gekühlte (20°C) konzentrierte Schwefelsäure geleitet. Das auf diese Weise erhaltene Rohgas enthält kein nachweisbares NH₃.

Die Neutralisation erfolgt derart, dass das HCN enthaltende Rohgas (Temperatur des Rohgases: 100°C) in einen 25 l Rührbehälter geleitet wird, in dem etwa 10 l einer 40 gew.-%igen wässrigen NaOH-Lösung vorgelegt werden. Dazu werden 30 l VE-Wasser zur weiteren Verdünnung gegeben. Während der Neutralisation bei 40°C (externe Kühlung) wird der Gehalt an freier NaOH ständig kontrolliert (Probenahme). Bei einem Überschuss von etwa 1,5 % NaOH wird die Gaseinleitung unterbrochen. Anschließend wird mit zusätzlichem VE-Wasser ein Cyanidgehalt von 50 Gew.-ppm eingestellt.

### 2 Herstellung von Blausäuresalz-Lösungen (erfindungsgemäß)

Prozessführung zur Herstellung des Blausäure enthaltenden Rohgases: Beispiel A1 (Formamid-Umsatz: 98,5 %; Blausäureselektivität: 93,2 %).

Die Zusammensetzung des in Beispiel A1 erhaltenen Rohgases ist wie folgt (in Gew.-%): 55,5 % HCN; 38,0 % Wasser; 1,5 % Formamid; 1,7 % NH₃; 2,9 % CO₂; 0,2 % H₂; 0,2 CO.

Zur NH₃-Entfernung wird das Rohgas durch eine gekühlte (20°C) konzentrierte Schwefelsäure geleitet. Das auf diese Weise erhaltene Rohgas enthält kein nachweisbares NH₃.

Die Neutralisation erfolgt derart, dass das HCN enthaltende Rohgas (Temperatur des Rohgases: 100 °C) in einen 25 l Rührbehälter geleitet wird, in dem etwa 10 l einer 40 gew.-%igen wässrigen NaOH-Lösung vorgelegt werden. Dazu werden erfindungsgemäß 30 l cyanidhaltiges Abwasser (Gehalt = 31 Gew.-ppm) aus dem Auslaugungsprozess von Golderz (Haufenlaugung, Heap-Leaching) zur weiteren Verdünnung gegeben. Während der Neutralisation bei 40°C (externe Kühlung) wird der Gehalt an freier NaOH ständig kontrolliert (Probenahme). Bei einem Überschuss von etwa 1,5 % NaOH wird die Gaseinleitung unterbrochen. Anschließend wird mit cyanidhaltigem Abwasser ein Cyanidgehalt von 50 Gew.-ppm eingestellt

### Säulenversuche:

Als Modell der industriell durchgeführten Haufenlaugung dienen Säulenversuche, bei denen repräsentative Proben des goldhaltigen Gesteins in Säulen gepackt und mit alkalischer Cyanid-Lösung ausgelaugt werden.

Die Laugung erfolgt jeweils über 30 Tage bei einer Flussrate von 180 ml/Tag kg Erz.

Die Säulen haben einen Innendurchmesser von 12 cm, eine Länge von 120 cm und sind jeweils mit 25 kg Erz befüllt. Das Erz wird auf eine Partikelgröße x von 0,5 ≤ x≤ 2,5 cm gemahlen und gesiebt. Der Goldgehalt beträgt jeweils 0,5-1,5 g/t Gestein. Es werden Erze der Minen Minera Yanacocha (Versuchsnummern 1a und 2a) und Minera Nueva California (Versuchsnummern 1b und 2b), Carretea Mancos km15, Distrito de Mancos, Provincia Yungay, Ancash - Peru eingesetzt. Die Ergebnisse sind in Tabelle 1 zusammengefasst.

Die kumulierten %-Angaben zur Goldausbeute beziehen sich auf den Gesamtgoldgehalt der einzelnen Säulen. Der Goldgehalt wirde mittels ICP-OES (Inductively coupled Plasma-Optical Emission Spectroscopy) auf einem Vista MPX der Firma Varian, Inc., Darmstadt bestimmt.

**Tabelle 1**

| Vers.-Nr.: | Verdünnungsmittel | Cyanid-Konzentration (Gew.-ppm) | Au-Ausbeute (%) | Abwasser (Liter) nach A-Kohle | Golderz |
|---|---|---|---|---|---|
| 1a | VE-Wasser | 50 | 79 | 4,9 | Yanacocha |
| 1b | VE-Wasser | 50 | 78 | 4,9 | Nueva C |
| 2a | Cyanid-Abwasser | 50 | 79 | 0,6*) | Yanacocha |
| 2b | Cyanid-Abwasser | 50 | 81 | 0,6*) | Nueva C |

| | | | | | |
|---|---|---|---|---|---|
| *) Das Abwasser wird wie folgt erhalten. Der Ablauf der Säulenlaugung wird über 30 Tage gesammelt (5,2 l) und der gelöste goldhaltige Komplex wird durch einen Aktivkohle-Filter von der übrigen Flüssigkeit durch Absorbtion abgetrennt. Der Ablauf der Aktivkohle Filtration (4,9 l) enthält noch ca. 30 ppm Natriumcyanid. Dieses Abwasser wird zur Herstellung von NaCN-Lösung eingesetzt. Ein Teilstrom (0,6 l) wird durch frisches VE-Wasser ersetzt, um Aufpegelungen durch Schwermetalle etc. zu vermeiden. | | | | | |

## Patentansprüche

1. Verfahren zur Herstellung von Lösungen enthaltend Wasser und mindestens ein Metallcyanid, umfassend:
(i) Umsetzung von Blausäure oder Blausäure enthaltenden Gasen mit einer Lösung enthaltend mindestens ein Metallhydroxid und/oder Metalloxid und/oder Mischoxid und Wasser;
(ii) gegebenenfalls Verdünnen der in Schritt (i) erhaltenen Lösung mit Wasser; wobei in Schritt (i) mindestens ein Teil des Wassers aus Abwasser gewonnen wird, das in einem Verfahren zur Gewinnung von Edelmetallen aus edelmetallhaltigen Erzen durch Cyanidlaugerei als abgereichertes Abwasser anfällt.

2. Verfahren nach Anspruch 1, **dadurch gekennzeichnet, dass** das eingesetzte Abwasser von Feststoffen und/oder Schwermetallen geklärtes in der Cyanidlaugerei anfallendes abgereichertes Abwasser ist.

3. Verfahren nach Anspruch 1 oder 2, **dadurch gekennzeichnet, dass** das mindestens eine Metallhydroxid und/oder Metalloxid und/oder Mischoxid ein Alkalimetall- oder Erdalkalimetallhydroxid und/oder Erdalkalimetalloxid und/oder Erdalkalimetallmischoxid ist, bevorzugt Li-, Na-, K- oder Ca-hydroxid und/oder Ca-Oxid und/oder Ca-Mischoxid, besonders bevorzugt Na-Hydroxid oder Ca-Hydroxid und/oder Ca-Oxid und/oder Ca-Mischoxid.

## Claims

1. A process for the preparation of solutions comprising water and at least one metal cyanide, comprising:
(i) reaction of hydrogen cyanide or hydrogen cyanide-comprising gases with a solution comprising at least one metal hydroxide and/or metal oxide and/or mixed oxide and water;
(ii) if appropriate, dilution of the solution obtained in step (i) with water; in step (i) at least a part of the water being obtained from wastewater which occurs as depleted wastewater in a process for extracting noble metals from noble metal-containing ores by the cyanide process.

2. The process according to claim 1, wherein the wastewater used is depleled wastewater clarified to remove solids and/or heavy metals and occurring in the cyanide process.

3. The process according to claim 1 or 2, wherein the at least one metal hydroxide and/or metal oxide and/or mixed oxide is an alkali metal or alkaline earth metal hydroxide and/or alkaline earth metal oxide and/or alkaline earth metal mixed oxide, preferably Li, Na, K or Ca hydroxide and/or Ca oxide and/or Ca mixed oxide, particularly preferably Na hydroxide or Ca hydroxide and/or Ca oxide and/or Ca mixed oxide.

## Revendications

1. Procédé pour la préparation de solutions contenant de l'eau et au moins un cyanure métallique, comprenant :
(i) la mise en réaction d'acide cyanhydrique ou de gaz qui contiennent de l'acide cyanhydrique avec une solution contenant au moins un hydroxyde métallique et/ou un oxyde métallique et/ou un oxyde mixte et de l'eau ;
(ii) éventuellement dilution avec de l'eau de la solution obtenue dans l'étape (i), au moins une partie de l'eau dans l'étape (i) étant obtenue à partir d'eau résiduaire qui est produite en tant qu'eau résiduaire appauvrie dans un procédé pour l'extraction de métaux nobles par cyanuration à partir de minerais contenant des métaux nobles.

2. Procédé selon la revendication 1, **caractérisé en ce que** l'eau résiduaire utilisée est de l'eau résiduaire appauvrie, produite dans la cyanuration, séparée de solides et/ou de métaux lourds.

3. Procédé selon la revendication 1 ou 2, **caractérisé en ce que** ledit au moins un hydroxyde métallique et/ou oxyde métallique et/ou oxyde mixte est un hydroxyde de métal alcalin ou de métal alcalino-terreux et/ou un oxyde de métal alcalino-terreux et/ou un oxyde mixte de métal alcalino-terreux, de préférence l'hydroxyde de Li, Na, K ou Ca et/ou l'oxyde de Ca et/ou un oxyde mixte de Ca, de façon particulièrement préférée l'hydroxyde de Na ou l'hydroxyde de Ca et/ou l'oxyde de Ca et/ou un oxyde mixte de Ca.
